# EUROPEAN PATENT SPECIFICATION

(11) **EP 3 667 296 B1**
(45) Date of publication and mention of the grant of the patent: **01.02.2023**
(21) Application number: 19216248.5
(22) Date of filing: 13.12.2019
(51) Int. Cl.: G01N 21/3504, G01N 21/94, G01N 21/17

(54) **OPTICAL DETECTION SYSTEM AND METHOD**
OPTISCHES DETEKTIONSSYSTEM UND -VERFAHREN
SYSTÈME ET PROCÉDÉ DE DÉTECTION OPTIQUE

(30) Priority: 14.12.2018 US 201816220485
(43) Date of publication of application: 17.06.2020
(73) Proprietor: The Boeing Company, Chicago, IL 60606-2016 (US)
(72) Inventor: MEFFORD, David K, Chicago, IL Illinois 60606-2016 (US); SMITH, Robert A, Chicago, IL Illinois 60606-2016 (US); LOWELL, John R, Chicago, IL Illinois 60606-2016 (US)
(74) Representative: Boult Wade Tennant LLP

(56) References cited:
- EP-A1- 2 219 022
- WO-A1-2018/213212
- US-A1- 2013 265 568
- US-B1- 6 985 818

## Description

### Field of the Disclosure

The present disclosure is directed to systems and methods for chemical detection. More particularly, the present disclosure is directed to remote chemical detection systems and methods using optical absorption detection with a matched reference.

### Background

It is known in the art to detect certain constituents in a chemical cloud in the air by spectral analysis of the molecules making up the cloud. This type of chemical detection has many applications, including detecting natural gas leaks from underground pipes, chemical clouds from chemical spills, volatile organic vapor (VOC) from chemical processes, pollution from smoke stacks and the like, military chemical warfare agents, and other toxic gases present in the air. Typically, this type of spectral analysis of a chemical cloud is performed remotely, sometimes up to 10-20 km away, because the constituents in the cloud may be toxic, and thus a threat to health, or it may not be possible to directly detect the chemical cloud. The distance the detecting instrument has to be from the cloud for this remote type of passive sensing depends on the particular application, and different systems exist for different applications.

To perform this type of detection and analysis, a spectrometer, such as a Fourier transform infrared (FTIR) spectrometer, is directed towards the chemical agent cloud from a remote location, so that it passively receives emissions therefrom. Typical optical absorption detection systems operate independent of each other and rely on long optical path lengths to absorb enough of a species for detection. The spectral display generated by the spectrometer from the emissions provides emission lines and bands at certain wavelengths that is indicative of the atoms and molecules in the cloud. Because each material has its own spectral "fingerprint" representative of its molecules, the detected spectral display can be compared to a known "fingerprint" of a particular chemical to determine if that chemical exists in the cloud.

What is needed is an improved remote chemical detection system that addresses various problem of the conventional approaches.

US 6,985,818 B1, in accordance with its abstract, states a continuous monitoring method and system wherein a porous substrate or film is used. The air from the environment is drawn through a region of the porous substrate by a simple air pump and the substances in the air are deposited or chemically absorbed onto the surface of the substrate. The region of the substrate where the environmental air is drawn through is continuously monitored by an optical or spectrometric method. The substrate is in the form of a tape supplied by a feed reel in a reel-to-reel cartridge and taken up by a take-up reel as found in a film cartridge or a magnetic tape cartridge. The cartridge can be replaceable. A variety of materials may be employed as the substrate with an adequate surface area to effect accumulation of solid, liquid, aerosol, or gas phase compounds. An optical interrogation system is engineered such that the surface of the tape at the point where air from the environment is drawn through the substrate becomes the interaction region between the source output and the sample. As material from the environment accumulates in this region, the interaction of the source with the material is monitored by a suitable detector and supporting circuitry.

EP 2,219,022 A1, in accordance with its abstract, states a hydrocarbon concentration measuring apparatus, which, even when the concentration and composition of hydrocarbons contained in an object gas to be measured vary, can measure the concentration of the hydrocarbons with good response and good accuracy, and a hydrocarbon measuring method. Light with a waveband including a common absorption region, which is absorbed by a single or a plurality of chemical species, is applied to the object gas by an infrared irradiation equipment. The light applied to the object gas is detected with a line sensor. The absorbance in the common absorption region of the object gas is computed with an analyzer based on the detected light. The sum of concentrations of chemical species, which absorb light in the waveband in the common absorption region, in the single or plurality of chemical species contained in the object gas, is computed with the analyzer based on the absorbance.

US 2013/265568 A1, in accordance with its abstract, states a device and method for identifying solid and liquid materials use near-infrared transmission spectroscopy combined with multivariate calibration methods for analysis of the spectral data. Near-infrared transmission spectroscopy is employed within either the 700-1100nm of the 900-1700nm wavelength range to identify solid or liquid materials and determine whether they match specific known materials. Uses include identifying solid (including powdered) and liquid materials with a fast measurement cycle time of about 2 to 15 seconds and with a method that requires no sample preparation, as well as quantitative analysis to determine the concentration of one or more chemical components in a solid or liquid sample that consists of a mixture of components. A primary application of such analysis includes detection of counterfeit drug tablets, capsules and liquid medications.

WO 2018/213212 A1, in accordance with its abstract, states a system and process that scan a target area at a distance of 3-30m for one or more materials. Scanning is performed by a coherent transmit beam aimed with the help of a thermal camera. The active source of the beam is a supercontinuum (SC) laser. The transmitted source beam is modulated by a high-speed Fourier-transform spectrometer prior to interaction with the target. Target reflected source beam is detected by an infrared detector, along with a reference portion of the transmitted source beam, as a series of interferograms; passed through a digitizer for digitizing the interferograms; and processed to producing spectrograms, wherein the spectrograms are indicative of one or more materials on the target.

### SUMMARY

There is described herein an optical detection method comprising: directing an optical beam toward a target; receiving, at a linear detector array, the optical beam after it has been reflected or scattered from the target to form a reflected or scattered optical beam; obtaining a target spectrum from the reflected or scattered optical beam received from the target by the linear detector array; selecting a first test sensor from among a plurality of test sensors, wherein the first test sensor comprises a first test chemical, wherein each test sensor of the plurality of test sensors comprises: a nanomaterial that is impregnated with a different test chemical, and/or a capsule with a different test chemical; obtaining a first matched spectrum from the first test chemical; performing a first matched filter comparison of the target spectrum with the first matched spectrum, the first matched spectrum corresponding to a first matched filter; determining a first candidate chemical likely to be present in the target based on the first matched filter comparison; and providing an identification of the first candidate chemical as an output based on the determining.

There is also described herein an optical detection device comprising: a transmitter configured to direct an optical beam toward a target; a linear detector array configured: to receive the optical beam after it has been reflected or scattered from the target to form a reflected or scattered optical beam; and to obtain a target spectrum from the reflected or scattered optical beam received from the target by the linear detector array; and a plurality of test sensors, wherein each test sensor of the plurality of test sensors comprises: a nanomaterial that is impregnated with a different test chemical, and/or capsule with a different test chemical; wherein: the optical detection device is configured: to select a first test sensor from among the plurality of test sensors, and to obtain a matched spectrum from the test chemical of the selected test sensor; the linear detector array is configured to perform a matched filter comparison of the target spectrum with the matched spectrum, the matched spectrum corresponding to a matched filter; and the optical detection device further comprises: a hardware processor configured to determine a candidate chemical likely to be present in the target based on the matched filter comparison; and an output interface configured to provide an identification of the candidate chemical as an output based on the determining.

It is to be understood that the following detailed description is exemplary and explanatory only and is not restrictive of the claimed optical detection method and device.

### BRIEF DESCRIPTION OF THE DRAWINGS

The accompanying drawings, which are incorporated in and constitute a part of this specification, illustrate features of the present teachings and together with the description, serve to explain the principles of the present teachings.
FIG. 1 illustrates a block diagram of an optical detection system for chemical detection using a matched reference, according to an implementation.
FIG. 2 illustrates a schematic view of the block diagram of FIG. 1.
FIG. 3 shows an optical detection method 300, according to an implementation.
FIG. 4 shows an optical detection method 400, according to an implementation.
FIG. 5 shows an example spectrum 500 that can be produced by optical detection method 300, according to examples of the present disclosure.
FIG. 6 shows an example spectrum 600 that does not include a reference chemical.

It should be noted that some details of the figures have been simplified and are drawn to facilitate understanding rather than to maintain strict structural accuracy, detail, and scale.

### DESCRIPTION

Reference will now be made in detail to the present teachings, examples of which are illustrated in the accompanying drawings. In the drawings, like reference numerals have been used throughout to designate identical elements. In the following description, reference is made to the accompanying drawings that form a part thereof, and in which is shown by way of illustration specific examples of practicing the present teachings. The following description is, therefore, merely exemplary.

Generally speaking, features of the present disclosure provides for an optical detection system and method that uses a matched filter (a matched absorption filter) to improve the signal to noise ratio of an optical spectrum. This provides the ability to "pull' a chemical at a target location out of the background and compare a spectrum of the chemical with spectra from one or more test chemicals. The matched filter technique is used to enhance the optical detection of a low concentration compound mixed with a background via absorption by optically combining a matched spectrum without background. The matched spectrum is determined by a detection system and also serves as the source for releasing the test chemical that provides the matched spectrum. The optical detection system and method provides for (1) the use of chemical absorption features in a matched spectrum optically combined with a signature in the background, (2) the use of an orthogonal sensor to determine the chemical release, and (3) the use of the same orthogonal sensor type to generate the release of a test chemical for optical absorption detection. The optical detection system and method provides for an enhancement to matching gaseous outputs of chemicals, including but are not limited to TNT.

The optical detection system and method can use nanotube chemical sensors that can be heated to release a chemical bonded to the nanotubes so that an optical beam probes the released chemical. The optical detection system and method probes the environment and uses subtractive detection to determine a likely chemical in the environment. The nanotubes can include a functionalized surface with chemical bonds such that, when heated, the chemical is released. The optical beam is directed through the released gas and is analyzed to determine if the environment also has the chemical. The optical detection system and method can use optical spectroscopy of the chemical through either absorption or emission spectroscopy. The optical detection system and method can be used for any type of environmental monitoring, explosives detection, environmental issues at smokestacks, pollution monitors, or fire detection.

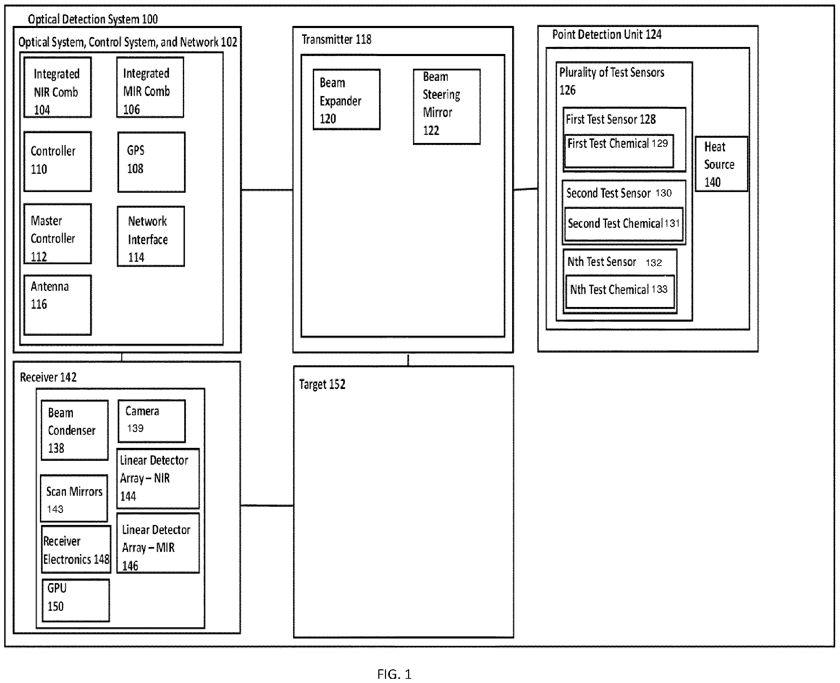

FIG. 1 illustrates a block diagram of an optical detection system 100 for chemical detection using a matched reference, according to an implementation. The optical detection system 100 comprises elements of an optical system, control system, and network 102 that are configured to produce and control an optical beam. The optical system of the optical detection system 100 can include an integrated near infrared ("NIR") frequency comb laser system 104 or an integrated mid infrared ("MIR") frequency comb laser system 106 (or both an integrated near infrared ("NIR") frequency comb laser system 104 and an integrated mid infrared ("MIR") frequency comb laser system 106, or more than one integrated near infrared ("NIR") frequency comb laser system 104 and/or more than one grated mid infrared ("MIR") frequency comb laser system 106. A frequency comb laser system 104, 106 is a laser source whose spectrum is made up of a series of discrete, equally spaced frequency lines. The frequency comb laser system 104, 106 can be implemented by a number of mechanisms, including periodic modulation (in amplitude and phase) of a continuous-wave laser, four wave mixing in nonlinear media, or stabilization of a pulse train generated by a mode-locked laser. In some examples, the lasers used for frequency-comb generation can be a Ti:sapphire solid-state lasers or Er:fiber lasers with repetition rates typically between 100 MHz and 1 GHz or even going as high as 10 GHz.

The optical system, control system, and network 102 includes a controller 110 that is configured to control the operation of the integrated NIR frequency comb laser system 104 and the integrated MIR frequency comb laser system 106. The optical system, control system, and network 102 also includes a network interface 114 and an antenna 116. A global positioning system ("GPS") module 108 can be in communication with the controller 110 and the master controller 112. The optical system, control system, and network 102 also comprises an output interface that is configured to provide an output for a user and/or to be provided to a computer system over a network by the network interface 114 and the antenna 116.

The optical detection system 100 also comprises a transmitter 118. The transmitter 118 is configured to direct an optical beam from the NIR frequency comb laser system 104 and/or the MIR frequency comb laser system 106 towards a target 152. The transmitter 118 can comprise a beam expander 120 that is configured to expand the optical beam, and the transmitter 118 can comprise a beam steering mirror 122 that is configured to steer the optical beam. The optical beam steered towards and received by the target 152 can comprise a plurality of spatially displaced optical beams.

The optical detection system 100 also comprises a point detection unit 124 that is configured to select a test sensor from among a plurality of test sensors 126. An optical beam received from the test sensor is compared with an optical beam received from the target 152. The plurality of test sensors 126 includes a first test sensor 128, a second test sensor 130, ..., an nth test sensor 132. The first test sensor 128 comprises a first test chemical 129, the second test sensor 130 comprises a second test chemical 131, and the nth test sensor 132 comprises an nth test chemical 133. Each of the plurality of test sensors 126 comprises a nanomaterial that is impregnated with a different one of the test chemicals. Each of the plurality of test sensors 126 can comprise a capsule with a different one of the test chemicals. The point detection unit 124 can also include a heat source 134 configured to heat one or more of the plurality of test sensors 126 to a temperature sufficient to release the respective test chemicals from the one or more of the plurality of test sensors 126 . In some examples, a single walled carbon nanotube material is used to mimic a natural receptor for molecular recognition, such as for recognition and hence detection of hydrogen sulfide, sulfur mustard or any number of other chemical agents with sensitivity levels of 10 ppm or less.

A receiver 142 is configured to receive a portion of the optical beams that are reflected or scattered from the target 152. The receiver 142 comprises a camera 139 and a beam focusing device, namely a beam condenser 138, that is configured to operate in reverse to the beam expander 120 by focusing the plurality of scattered and/or reflected optical beams received from the target 152. The receiver 142 also comprises a NIR linear detector array 144 and/or a MIR linear detector array 146, whose outputs are received by the receiver electronics 148 and processed by a hardware processor, such as by a central processing unit ("CPU") or a graphics processor unit ("GPU") 150. The NIR linear detector array 144 and/or MIR the linear detector array 146 are configured to compare one or more spectra taken from an optical beam received from one or more of the test chemicals. For example, a first spectrum obtained from the optical beam received from the first test chemical 129 may be compared with a spectrum obtained from the reflected or scattered optical beam that was received from the target 152. The processor, such as the GPU 150, is configured to determine a chemical present in the target 152 based on the results of the comparison of the spectra.

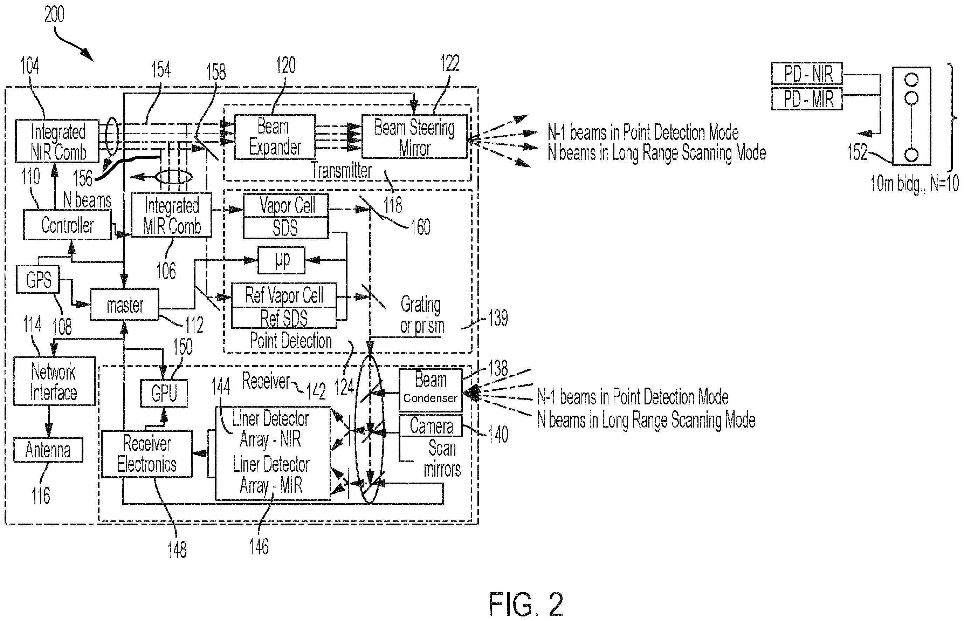

FIG. 2 illustrates a schematic view 200 of the optical detection system 100, showing additional details. The integrated NIR frequency comb laser system 104 and the integrated MIR frequency comb laser system 106 are configured to produce a first plurality of spatially displaced optical beams 154 and a second plurality of spatially displaced optical beams 156, respectively. A portion of the first plurality of spatially displaced optical beams 154 and the second plurality of spatially displaced optical beams 156 is directed to the point detection unit 124 by a first mirror 158. The optical beams from the point detection unit 124 is directed to the receiver 142 by a second mirror 160, which is combined with radiation received from the target 152 by a beam splitter and/or a grating or a prism 139 to be analyzed by the NIR linear detector array 144 and/or the MIR linear detector array 146, the receiver electronics 148, and the GPU 150.

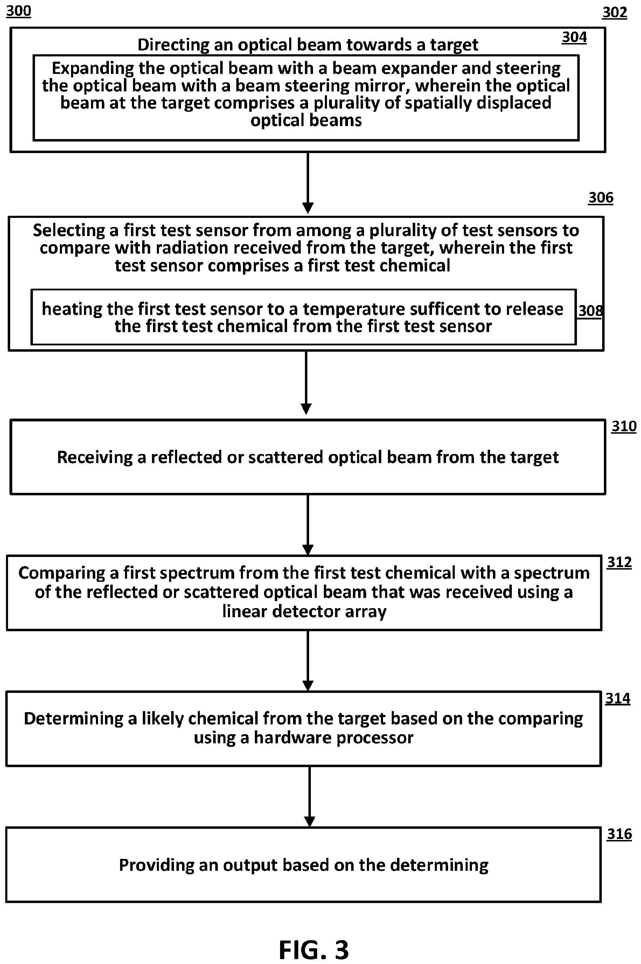

FIG. 3 shows an optical detection method 300, according to examples of the present disclosure. According to the claimed optical detection method, the optical detection method 300 begins by directing, at 302, the optical beam toward the target 152. In some examples, directing the optical beam further comprises expanding, at 304, the optical beam with the beam expander 120 and steering the optical beam with the beam steering mirror 122. The optical beam steered towards the target 152 comprises a plurality of spatially displaced optical beams.

The optical detection method 300 continues by selecting, at 306, the first test sensor 128 from among the plurality of test sensors 126. An optical beam received from the first test sensor 128 is compared with the portion of the optical beam radiation reflected or scattered from the target 152. In some examples, selecting the first test sensor 128 can further comprise heating, at 308, the first test sensor 128 to a temperature sufficient to release the first test chemical 129 from the first test sensor 128. The first test sensor 128 comprises the first test chemical 129, the second sensor 132 comprises the second test chemical 131, ..., the nth test sensor 136 comprises the nth test chemical 133. Each of the plurality of test sensors 126 comprises a nanomaterial that is impregnated with a different test chemical and/or a capsule with a different chemical test.

The optical detection method 300 continues by receiving, at 310, the reflected or scattered optical beam from the target 152.

The optical detection method 300 continues by comparing, at 312, the first spectrum obtained from the first test chemical 129 with the spectrum obtained from the reflected or scattered optical beam that was received by the linear detector array 144, 146.

For example, the local atmosphere of one of the plurality of test sensors 126 can be interrogated by the transmitter 118. The test sensor, such as the first test sensor 128, is heated and the first test chemical 129 is released into the local atmosphere ingested by the test sensor. The released first test chemical 129 and local atmosphere can then be interrogated by the portion of the optical beam separated by the first mirror 158 from the transmitter 118 as it passes through the point detection unit 124.

The optical detection method 300 continues by using a hardware processor to determine (identify), at 314, a chemical present at the target 152 based on comparing the spectra at 312.

The optical detection method 300 continues by providing, at 316, an output based on the determining.

Typically a chemical spectrum (a spectrum collected from a chemical sample) is defined by its absorption features as a function of frequency. A chemical spectrum will have specific absorption features at different frequencies associated with the constituent chemicals that make up the chemical sample. The specific absorption features at different frequencies is a signature of each constituent chemical. By identifying the specific absorption features, a database may be generated in a controlled environment with the optical detection system 100. However, the specific features of a chemical may be masked or hidden amongst the background signal in a spectrum. A method of increasing the specific absorption features of the chemical is provided by the present disclosure as will now be described.

As described above, the optical beams received from the target 152 and the selected one of the plurality of test sensors 126 are combined. This means that the spectrum from the target 152 is effectively added to the spectrum from the selected one of the plurality of test sensors 126 to form a combined spectrum. As the spectrum from the selected one of the plurality of test sensors 126 acts as a matched filter, this addition of the spectra causes the specific absorption features in the combined spectrum to increase relative to the background, i.e. there is an improvement in the signal to background ratio. A processing method can then be used to correlate the combined spectrum to spectra stored in a database such that an identification of a chemical in the target 152 may be made with the highest confidence.

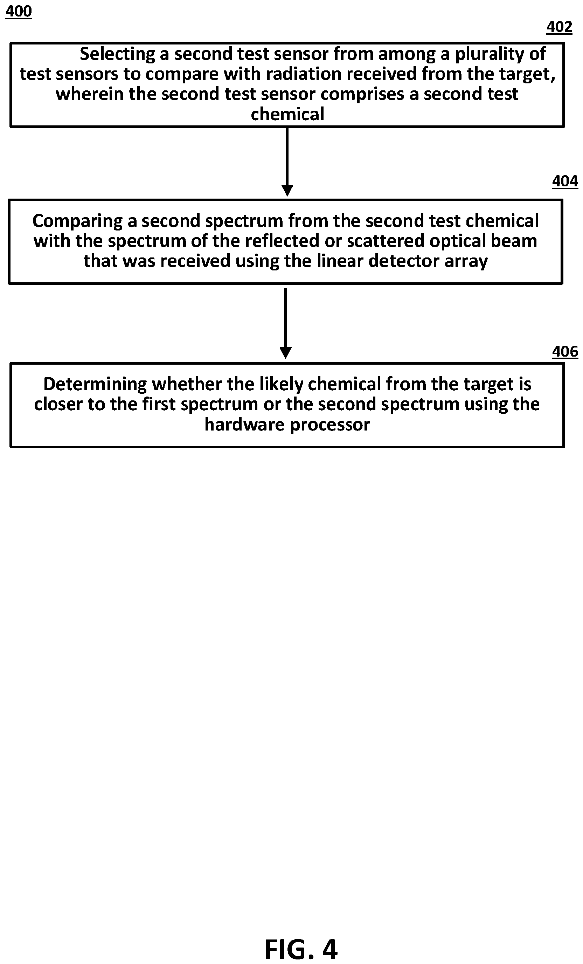

FIG. 4 shows an optical detection method 400, according to examples of the present disclosure. The method 400 is a continuation of the method 300. The method 400 begins by selecting, at 402, a second test sensor 130 from among the plurality of test sensors 126. An optical beam received from the second test sensor 130 for a comparison with the reflected or scattered optical beam received from the target 152. The second test sensor 130 comprises the second test chemical 131.

The method 400 continues by comparing, 404, a second spectrum obtained from the optical beam received from the second test chemical 131 with the spectrum obtained from the reflected or scattered optical beam that was received from the target 152 using the linear detector array 144, 146.

The method 400 continues by using the hardware processor to determine, at 406, whether the specific absorption features of the chemical present at the target 152 that appear in the spectrum obtained from the target 152 is more similar to features seen in the first spectrum obtained from the first test sensor 128 or seen in the second spectrum obtained from the second test sensor 130.

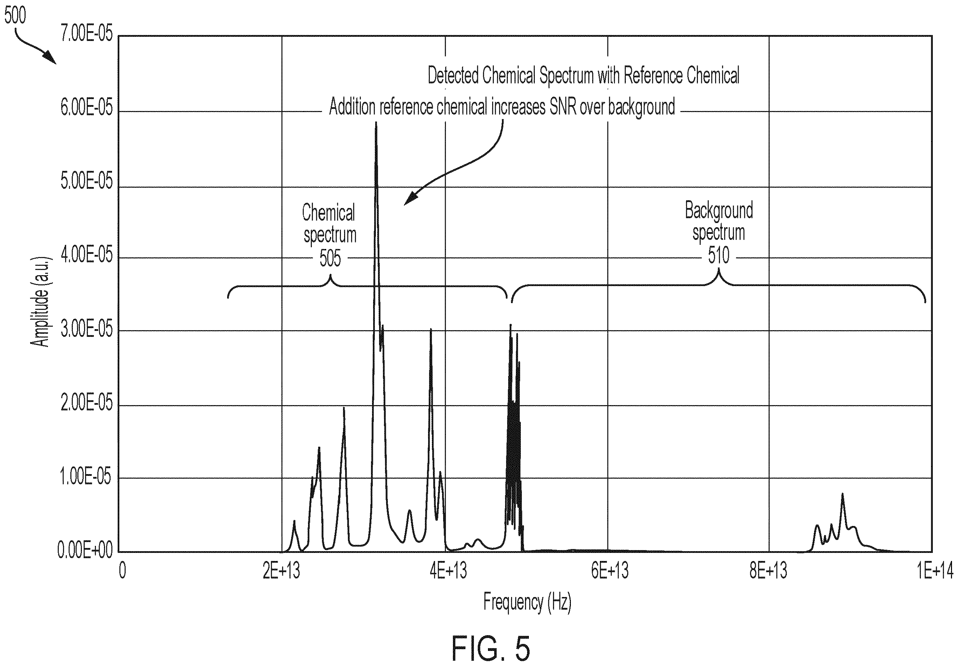

FIG. 5 shows an example combined spectrum 500 that can be produced by the optical detection method 300, according to examples of the present disclosure. The spectrum 500 shows a first portion comprising the chemical spectrum 505 that includes specific absorption features of a test chemical, such as the first test chemical 129 or the second test chemical 131, and which shows the increased signal-to-noise ratio relative to the background. The spectrum 500 also shows a second portion comprising the background spectrum 510.

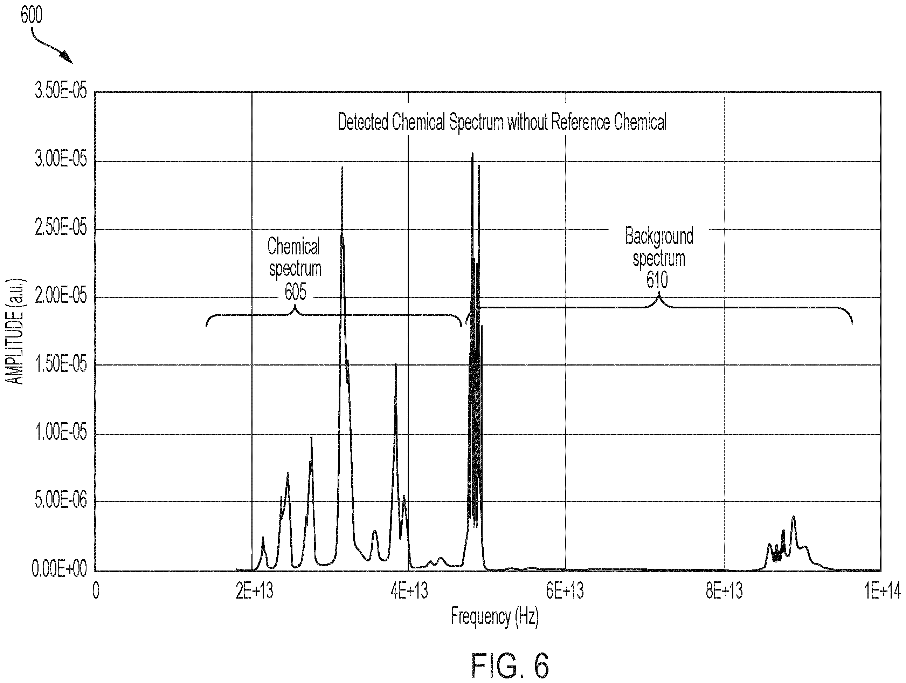

By comparison, FIG. 6 shows an example spectrum 600 taken from a target 152 and that has not been combined with a spectrum obtained from a test chemical, such as the first test chemical 129 or the second test chemical 131. The spectrum 600 shows a first portion comprising the chemical spectrum 505 and a second portion comprising the background spectrum 610.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all sub-ranges subsumed therein.

While the present teachings have been illustrated with respect to one or more implementations, alterations and/or modifications can be made to the illustrated examples without departing from the scope of the appended claims.

Further, in the discussion and claims herein, the term "about" indicates that the value listed may be somewhat altered, as long as the alteration does not result in nonconformance of the process or structure to the intended purpose described herein.

Finally, "exemplary" indicates the description is used as an example, rather than implying that it is an ideal. It will be appreciated that variants of the above-disclosed and other features and functions, or alternatives thereof, may be combined into many other different systems or applications. Various modifications, variations, or improvements may be made by those skilled in the art within the scope of the following claims.

## Claims

1. An optical detection method comprising:
directing (302) an optical beam toward a target (152);
receiving (310), at a linear detector array (144, 146), the optical beam after it has been reflected or scattered from the target (152) to form a reflected or scattered optical beam;
obtaining a target spectrum from the reflected or scattered optical beam received from the target (152) by the linear detector array (144, 146);
selecting (306) a first test sensor (128) from among a plurality of test sensors (128, 130, 132), wherein the first test sensor (128) comprises a first test chemical (129), wherein each test sensor of the plurality of test sensors comprises:
a nanomaterial that is impregnated with a different test chemical (129, 131, 133),
and/or
a capsule with a different test chemical (129, 131, 133);
obtaining a first matched spectrum from the first test chemical (129);
performing (312) a first matched filter comparison of the target spectrum with the first matched spectrum, the first matched spectrum corresponding to a first matched filter;
determining (314) a first candidate chemical likely to be present in the target (152) based on the first matched filter comparison; and
providing (316) an identification of the first candidate chemical as an output based on the determining.

2. The optical detection method of claim 1, wherein obtaining the first matched spectrum from the first test chemical (129) comprises:
directing (302) a further optical beam toward the first test sensor (128);
receiving (310), at the linear detector array (144, 146), the further optical beam after it has been reflected or scattered from the first test sensor (128) to form a further reflected or scattered optical beam; and
obtaining the first matched spectrum from the further reflected or scattered optical beam received by the linear detector array (144, 146).

3. The optical detection method of claim 2, further comprising heating (308) the first test sensor (128) to a temperature sufficient to release the first test chemical (129) from the first test sensor (128) prior to directing (302) the further optical beam toward the first test sensor (128).

4. The optical detection method of any of claims 1-3, wherein the first test sensor (128) is selected based on user input provided by a user after an initial assessment of the first candidate chemical likely to be present in the target (152).

5. The optical detection method of any of claims 1-4, further comprising:
selecting (402) a second test sensor (130) from among the plurality of test sensors, wherein the second test sensor (130) comprises a second test chemical (131);
obtaining a second matched spectrum from the second test chemical (131);
performing (404) a second matched filter comparison of the target spectrum with the second matched spectrum, the second matched spectrum corresponding to a second matched filter;
determining (314) a second candidate chemical likely to be present in the target (152) based on the second matched filter comparison; and
determining (406) whether the first candidate chemical identified using the first matched spectrum or the second candidate chemical identified using the second matched spectrum is more likely to be present in the target.

6. The optical detection method of claim 5, wherein obtaining the second matched spectrum from the second test chemical (131) comprises:
directing (302) a still further optical beam toward the second test sensor (130);
receiving (310), at the linear detector array (144, 146), the still further optical beam after it has been reflected or scattered from the second test sensor (130) to form a still further reflected or scattered optical beam; and
obtaining the second matched spectrum from the still further reflected or scattered optical beam received by the linear detector array (144, 146).

7. The optical detection method of any of claims 1-6, further comprising expanding (304) the optical beam with a beam expander (120) to form an expanded optical beam, and steering the expanded optical beam towards the target (152) with a beam steering mirror (122), such that the expanded optical beam comprises a plurality of spatially displaced optical beams at the target (152).

8. The optical detection method of any of claims 1-7, comprising:
selecting (402) all test sensors from among the plurality of test sensors, wherein each test sensor (132) comprises a different test chemical;
obtaining a matched spectrum from each of the test chemicals;
performing (404) further matched filter comparisons of the target spectrum with each matched spectrum obtained from each test chemical, each matched spectrum corresponding to a matched filter; and
determining (314) a candidate chemical likely to be present in the target (152) for each of the further matched filter comparisons; and
determining (406) the candidate chemical most likely to be present in the target (152) from the determined candidate chemicals prior to providing the output.

9. The optical detection method of any previous claim, wherein the selecting (306) further comprises heating (308) the first test sensor (128) to a temperature sufficient to release the first test chemical (129) from the first test sensor (128).

10. The optical detection method of any previous claim, wherein the first test sensor (128) is selected based on an initial assessment of the likely chemical from the target (152).

11. An optical detection device comprising:
a transmitter (118) configured to direct an optical beam toward a target (152);
a linear detector array (144, 146) configured:
to receive the optical beam after it has been reflected or scattered from the target (152) to form a reflected or scattered optical beam; and
to obtain a target spectrum from the reflected or scattered optical beam received from the target (152) by the linear detector array (144, 146); and
a plurality of test sensors (128, 130, 132), wherein each test sensor of the plurality of test sensors comprises:
a nanomaterial that is impregnated with a different test chemical (129, 131, 133),
and/or
a capsule with a different test chemical (129, 131, 133);
wherein:
the optical detection device is configured:
to select a test sensor (128, 130, 132) from among the plurality of test sensors,
and
to obtain a matched spectrum from the test chemical (129, 131, 133) of the selected test sensor;
the linear detector array (144, 146) is configured to perform a matched filter comparison of the target spectrum with the matched spectrum, the matched spectrum corresponding to a matched filter; and
the optical detection device further comprises:
a hardware processor (150) configured to determine a candidate chemical likely to be present in the target (152) based on the matched filter comparison; and
an output interface configured to provide an identification of the candidate chemical as an output based on the determining.

12. The optical detection device of claim 11, further comprising a heat source (140) configured to heat the test sensor (128, 130, 132) to a temperature sufficient to release the test chemical (129, 131, 133) from the test sensor (128, 130, 132).

13. The optical detection device of claim 11 or claim 12, further comprising a beam expander (120) configured to expand the optical beam to form an expanded optical beam and a beam steering mirror (122) configured to steer the expanded optical beam, such that the optical beam comprises a plurality of spatially displaced optical beams at the target (152).

14. The optical detection device of any of claims 11-13, further comprising an integrated near infrared comb device (104) and/or an integrated mid-infrared comb device (106), where an optical output of the integrated near infrared comb device (104) and the integrated mid-infrared comb device (106) is provided to the transmitter (118).

15. The optical detection device of any of claims 11-14, wherein the linear detector array (144, 146) further comprises a beam focusing device (138) configured to receive the reflected or scattered optical beam from the target (152), and one or more scan mirrors (143) configured to provide one or more scanned optical beams to the linear detector array (144, 146).

## Patentansprüche

1. Optisches Nachweisverfahren, umfassend:
Richten (302) eines optischen Strahls auf ein Ziel (152);
Empfangen (310) des optischen Strahls an einer linearen Detektoranordnung (144, 146), nachdem er von dem Ziel (152) reflektiert oder gestreut wurde, um einen reflektierten oder gestreuten optischen Strahl zu bilden;
Gewinnen eines Zielspektrums aus dem von dem Ziel (152) durch die lineare Detektoranordnung (144, 146) empfangenen reflektierten oder gestreuten optischen Strahl;
Auswählen (306) eines ersten Testsensors (128) aus einer Mehrzahl von Testsensoren (128, 130, 132), wobei der erste Testsensor (128) eine erste Testchemikalie (129) umfasst, wobei jeder Testsensor der Mehrzahl von Testsensoren umfasst:
ein Nanomaterial, das mit einer anderen Testchemikalie (129, 131, 133) imprägniert ist, und/oder
eine Kapsel mit einer anderen Testchemikalie (129, 131, 133);
Gewinnen eines ersten Abgleichspektrums von der ersten Testchemikalie (129);
Durchführen (312) eines ersten Abgleichfiltervergleichs des Zielspektrums mit dem ersten Abgleichspektrum, wobei das erste Abgleichspektrum einem ersten Abgleichfilter entspricht;
Bestimmen (314) einer wahrscheinlich in dem Ziel (152) vorhandenen ersten Kandidatenchemikalie auf der Grundlage des ersten Abgleichfiltervergleichs ; und
Bereitstellen (316) einer Identifikation des ersten chemischen Kandidaten als auf dem Bestimmen basierende Ausgabe.

2. Optisches Nachweisverfahren nach Anspruch 1, bei dem das Gewinnen des ersten Abgleichspektrums von der ersten Testchemikalie (129) umfasst:
Richten (302) eines weiteren optischen Strahls auf den ersten Testsensor (128);
Empfangen (310) des weiteren optischen Strahls an der linearen Detektoranordnung (144, 146), nachdem er von dem ersten Testsensor (128) reflektiert oder gestreut wurde, um einen weiteren reflektierten oder gestreuten optischen Strahl zu bilden; und
Gewinnen des ersten Abgleichspektrums aus dem von der linearen Detektoranordnung (144, 146) empfangenen weiteren reflektierten oder gestreuten optischen Strahl.

3. Optisches Nachweisverfahren nach Anspruch 2, das ferner das Erwärmen (308) des ersten Testsensors (128) auf eine Temperatur umfasst, die ausreicht, um die erste Testchemikalie (129) aus dem ersten Testsensor (128) freizusetzen, bevor der weitere optische Strahl auf den ersten Testsensor (128) gerichtet wird (302).

4. Optisches Nachweisverfahren nach einem der Ansprüche 1-3, bei dem der erste Testsensor (128) auf der Grundlage von Benutzereingaben ausgewählt wird, die von einem Benutzer nach einer anfänglichen Einschätzung, welche erste Kandidatenchemikalie wahrscheinlich im Ziel (152) vorhanden ist, bereitgestellt werden.

5. Optisches Nachweisverfahren nach einem der Ansprüche 1-4, das ferner umfasst:
Auswählen (402) eines zweiten Testsensors (130) aus der Mehrzahl von Testsensoren, wobei der zweite Testsensor (130) eine zweite Testchemikalie (131) umfasst;
Gewinnen eines zweiten Abgleichspektrums von der zweiten Testchemikalie (131);
Durchführen (404) eines zweiten Abgleichfiltervergleichs des Zielspektrums mit dem zweiten Abgleichspektrum, wobei das zweite Abgleichspektrum einem zweiten Abgleichfilter entspricht;
Bestimmen (314) einer zweiten Kandidatenchemikalie, die wahrscheinlich in dem Ziel (152) vorhanden ist, basierend auf dem zweiten Abgleichfiltervergleich; und
Bestimmen (406), ob die unter Verwendung des ersten Abgleichspektrums identifizierte erste Kandidatenchemikalie oder die unter Verwendung des zweiten Abgleichspektrums identifizierte zweite Kandidatenchemikalie mit größerer Wahrscheinlichkeit in dem Ziel vorhanden ist.

6. Optisches Nachweisverfahren nach Anspruch 5, bei dem das Gewinnen des zweiten Abgleichspektrums von der zweiten Testchemikalie (131) umfasst:
Richten (302) noch eines weiteren optischen Strahls auf den zweiten Testsensor (130);
Empfangen (310) des noch weiteren optischen Strahls an der linearen Detektoranordnung (144, 146), nachdem er von dem zweiten Testsensor (130) reflektiert oder gestreut wurde, um noch einen weiteren reflektierten oder gestreuten optischen Strahl zu bilden; und
Gewinnen des zweiten Abgleichspektrums aus dem von der linearen Detektoranordnung (144, 146) empfangenen noch weiteren reflektierten oder gestreuten optischen Strahl.

7. Optisches Nachweisverfahren nach einem der Ansprüche 1 bis 6, ferner umfassend das Aufweiten (304) des optischen Strahls mit einem Strahlaufweiter (120), um einen aufgeweiteten optischen Strahl zu bilden, und das Lenken des aufgeweiteten optischen Strahls in Richtung des Ziels (152) mit einem Strahllenkspiegel (122), so dass der aufgeweitete optische Strahl am Ziel (152) eine Mehrzahl von räumlich versetzten optischen Strahlen umfasst.

8. Optisches Nachweisverfahren nach einem der Ansprüche 1-7, umfassend:
Auswählen (402) aller Testsensoren aus der Mehrzahl von Testsensoren, wobei jeder Testsensor (132) eine andere Testchemikalie umfasst;
Gewinnen eines Abgleichspektrums von jeder der Testchemikalien;
Durchführen (404) von weiteren Abgleichfiltervergleichen des Zielspektrums mit jedem von jeder Testchemikalie erhaltenen Abgleichspektrum, wobei jedes Abgleichspektrum einem Abgleichfilter entspricht; und
Bestimmen (314) einer wahrscheinlich in dem Ziel (152) vorhandenen Kandidatenchemikalie für jeden der weiteren Abgleichfiltervergleiche; und
Bestimmen (406) der am wahrscheinlichsten in dem Ziel (152) vorhandenen Kandidatenchemikalie aus den bestimmten Kandidatenchemikalien, bevor die Ausgabe bereitgestellt wird.

9. Optisches Nachweisverfahren nach einem der vorhergehenden Ansprüche, bei dem das Auswählen (306) ferner das Erhitzen (308) des ersten Testsensors (128) auf eine Temperatur umfasst, die ausreicht, um die erste Testchemikalie (129) aus dem ersten Testsensor (128) freizusetzen.

10. Optisches Nachweisverfahren nach einem der vorhergehenden Ansprüche, wobei der erste Testsensor (128) auf der Grundlage einer anfänglichen Bewertung der wahrscheinlichen Chemikalie aus dem Ziel (152) ausgewählt wird.

11. Optische Nachweisvorrichtung, umfassend:
einen Sender (118), der konfiguriert ist, um einen optischen Strahl auf ein Ziel (152) zu richten;
eine lineare Detektoranordnung (144, 146), konfiguriert zum:
Empfangen des optischen Strahls, nachdem er von dem Ziel (152) reflektiert oder gestreut wurde, um einen reflektierten oder gestreuten optischen Strahl zu bilden; und
Gewinnen eines Zielspektrums aus dem von dem Ziel (152) durch die lineare Detektoranordnung (144, 146) empfangenen reflektierten oder gestreuten optischen Strahl; und
eine Mehrzahl von Testsensoren (128, 130, 132), wobei jeder Testsensor der Mehrzahl von Testsensoren umfasst:
ein Nanomaterial, das mit einer anderen Testchemikalie (129, 131, 133) imprägniert ist, und/oder
eine Kapsel mit einer anderen Testchemikalie (129, 131, 133); wobei:
die optische Nachweiseinrichtung konfiguriert ist zum:
Auswählen eines Testsensors (128, 130, 132) aus der Mehrzahl von Testsensoren, und
Gewinnen eines Abgleichspektrums von der Testchemikalie (129, 131, 133) des ausgewählten Testsensors;
die lineare Detektoranordnung (144, 146) konfiguriert ist zum Durchführen eines Abgleichfiltervergleichs des Zielspektrums mit dem Abgleichspektrum, wobei das Abgleichspektrum einem Abgleichfilter entspricht; und
die optische Erfassungsvorrichtung ferner umfasst:
einen Hardware-Prozessor (150), der so konfiguriert ist zum Bestimmen einer wahrscheinlich in dem Ziel vorhandenen Kandidatenchemikalie (152) auf der Grundlage des Abgleichfiltervergleichs; und
eine Ausgabeschnittstelle, die konfiguriert zum Bereitstellen einer Identifikation der Kandidatenchemikalie als eine auf dem Bestimmen basierende Ausgabe.

12. Optische Nachweisvorrichtung nach Anspruch 11, die ferner eine Wärmequelle (140) umfasst, die konfiguriert ist zum Erwärmen des Testsensors (128, 130, 132) auf eine Temperatur, die ausreicht, um die Testchemikalie (129, 131, 133) aus dem Testsensor (128, 130, 132) freizusetzen.

13. Optische Nachweisvorrichtung nach Anspruch 11 oder Anspruch 12, ferner mit einem Strahlaufweiter (120), der konfiguriert ist, um den optischen Strahl aufzuweiten, um einen aufgeweiteten optischen Strahl zu bilden, und einem Strahllenkspiegel (122), der konfiguriert ist, um den aufgeweiteten optischen Strahl zu lenken, so dass der optische Strahl am Ziel (152) eine Mehrzahl von räumlich versetzten optischen Strahlen umfasst.

14. Optische Nachweisvorrichtung nach einem der Ansprüche 11 bis 13, ferner mit einer integrierten Nahinfrarot-Kammvorrichtung (104) und/oder einer integrierten Mittelinfrarot-Kammvorrichtung (106), wobei eine optische Ausgabe der integrierten Nahinfrarot-Kammvorrichtung (104) und der integrierten Mittelinfrarot-Kammvorrichtung (106) dem Sender (118) zugeführt wird.

15. Optische Nachweisvorrichtung nach einem der Ansprüche 11 bis 14, bei der die lineare Detektoranordnung (144, 146) ferner eine Strahlfokussierungsvorrichtung (138), die konfiguriert ist, um den reflektierten oder gestreuten optischen Strahl von dem Ziel (152) zu empfangen, und einen oder mehrere Abtastspiegel (143) umfasst, die konfiguriert sind, um einen oder mehrere abgetastete optische Strahlen an die lineare Detektoranordnung (144, 146) zu liefern.

## Revendications

1. Procédé de détection optique, comprenant les étapes consistant à :
diriger (302) un faisceau optique vers une cible (152) ;
recevoir (310), au niveau d'un réseau de détecteurs linéaire (144, 146), le faisceau optique après qu'il ait été réfléchi ou diffusé à partir de la cible (152) pour former un faisceau optique réfléchi ou diffusé ;
obtenir un spectre cible à partir du faisceau optique réfléchi ou diffusé reçu en provenance de la cible (152) par le réseau de détecteurs linéaire (144, 146) ;
sélectionner (306) un premier capteur de test (128) parmi une pluralité de capteurs de test (128, 130, 132), dans lequel le premier capteur de test (128) comprend un premier produit chimique de test (129), dans lequel chaque capteur de test de la pluralité de capteurs de test comprend :
un nanomatériau qui est imprégné d'un produit chimique de test différent (129, 131, 133), et/ou
une capsule avec un produit chimique de test différent (129, 131, 133) ;
obtenir un premier spectre apparié à partir du premier produit chimique de test (129) ;
effectuer (312) une première comparaison de filtre apparié du spectre cible avec le premier spectre apparié, le premier spectre apparié correspondant à un premier filtre apparié ;
déterminer (314) un premier produit chimique candidat susceptible d'être présent dans la cible (152) sur la base de la première comparaison de filtre apparié ; et
fournir (316) une identification du premier produit chimique candidat en tant que sortie sur la base de la détermination.

2. Procédé de détection optique selon la revendication 1, dans lequel l'obtention du premier spectre apparié à partir du premier produit chimique de test (129) comprend les étapes consistant à :
diriger (302) un faisceau optique supplémentaire vers le premier capteur de test (128) ;
recevoir (310), au niveau du réseau de détecteurs linéaire (144, 146), le faisceau optique supplémentaire après qu'il ait été réfléchi ou diffusé depuis le premier capteur de test (128) afin de former un faisceau optique réfléchi ou diffusé supplémentaire ; et
obtenir le premier spectre apparié à partir du faisceau optique réfléchi ou diffusé supplémentaire reçu par le réseau de détecteurs linéaire (144, 146).

3. Procédé de détection optique selon la revendication 2, comprenant en outre un chauffage (308) du premier capteur de test (128) à une température suffisante pour libérer le premier produit chimique de test (129) à partir du premier capteur de test (128) avant de diriger (302) le faisceau optique supplémentaire vers le premier capteur de test (128).

4. Procédé de détection optique selon l'une quelconque des revendications 1 à 3, dans lequel le premier capteur de test (128) est sélectionné sur la base d'une entrée d'utilisateur fournie par un utilisateur après une évaluation initiale du premier produit chimique candidat susceptible d'être présent dans la cible (152).

5. Procédé de détection optique selon l'une quelconque des revendications 1 à 4, comprenant en outre les étapes consistant à :
sélectionner (402) un second capteur de test (130) parmi la pluralité de capteurs de test, dans lequel le second capteur de test (130) comprend un second produit chimique de test (131) ;
obtenir un second spectre apparié à partir du second produit chimique de test (131) ;
effectuer (404) une seconde comparaison de filtre apparié du spectre cible avec le second spectre apparié, le second spectre apparié correspondant à un second filtre apparié ;
déterminer (314) un second produit chimique candidat susceptible d'être présent dans la cible (152) sur la base de la seconde comparaison de filtre apparié ; et
déterminer (406) si le premier produit chimique candidat identifié à l'aide du premier spectre apparié ou le second produit chimique candidat identifié à l'aide du second spectre apparié est plus susceptible d'être présent dans la cible.

6. Procédé de détection optique selon la revendication 5, dans lequel l'obtention du second spectre apparié à partir du second produit chimique de test (131) comprend les étapes consistant à :
diriger (302) un faisceau optique encore supplémentaire vers le second capteur de test (130) ;
recevoir (310), au niveau du réseau de détecteurs linéaire (144, 146), le faisceau optique supplémentaire après qu'il ait été réfléchi ou diffusé à partir du second capteur de test (130) pour former un faisceau optique réfléchi ou diffusé supplémentaire ; et
obtenir le second spectre apparié à partir du faisceau optique réfléchi ou dispersé encore supplémentaire reçu par le réseau de détecteurs linéaire (144, 146).

7. Procédé de détection optique selon l'une quelconque des revendications 1 à 6, comprenant en outre une dilatation (304) du faisceau optique avec un dilatateur de faisceau (120) pour former un faisceau optique dilaté, et une orientation du faisceau optique dilaté vers la cible (152) avec un miroir d'orientation de faisceau (122), de telle sorte que le faisceau optique dilaté comprend une pluralité de faisceaux optiques déplacés spatialement au niveau de la cible (152).

8. Procédé de détection optique selon l'une quelconque des revendications 1 à 7, comprenant en les étapes consistant à :
sélectionner (402) tous les capteurs de test parmi la pluralité de capteurs de test, dans lequel chaque capteur de test (132) comprend un produit chimique de test différent ;
obtenir un spectre apparié à partir de chacun des produits chimiques de test ;
effectuer (404) des comparaisons de filtre apparié supplémentaires du spectre cible avec chaque spectre apparié obtenu à partir de chaque produit chimique de test, chaque spectre apparié correspondant à un filtre apparié ; et
déterminer (314) un produit chimique candidat susceptible d'être présent dans la cible (152) pour chacune des comparaisons de filtre apparié ultérieures ; et
déterminer (406) le produit chimique candidat le plus susceptible d'être présent dans la cible (152) à partir des produits chimiques candidats déterminés avant de fournir la sortie.

9. Procédé de détection optique selon l'une quelconque des revendications précédentes, dans lequel la sélection (306) comprend en outre un chauffage (308) du premier capteur de test (128) jusqu'à une température suffisante pour libérer le premier produit chimique de test (129) à partir du premier capteur de test (128).

10. Procédé de détection optique selon l'une quelconque des revendications précédentes, dans lequel le premier capteur de test (128) est sélectionné sur la base d'une évaluation initiale du produit chimique susceptible à partir de la cible (152).

11. Dispositif de détection optique comprenant :
un émetteur (118) configuré pour diriger un faisceau optique vers une cible (152) ;
un réseau de détecteurs linéaire (144, 146) configuré :
pour recevoir le faisceau optique après qu'il ait été réfléchi ou diffusé à partir de la cible (152) afin de former un faisceau optique réfléchi ou diffusé ; et
pour obtenir un spectre cible à partir du faisceau optique réfléchi ou diffusé reçu en provenance de la cible (152) par le réseau de détecteurs linéaire (144, 146) ; et
une pluralité de capteurs de test (128, 130, 132), dans lequel chaque capteur de test de la pluralité de capteurs de test comprend :
un nanomatériau qui est imprégné d'un produit chimique de test différent (129, 131, 133), et/ou
une capsule avec un produit chimique de test différent (129, 131, 133) ;
dans lequel :
le dispositif de détection optique est configuré :
pour sélectionner un capteur de test (128, 130, 132) parmi la pluralité de capteurs de test, et
pour obtenir un spectre apparié à partir du produit chimique de test (129, 131, 133) du capteur de test sélectionné ;
le réseau de détecteurs linéaire (144, 146) est configuré pour effectuer une comparaison de filtre apparié du spectre cible avec le spectre apparié, le spectre apparié correspondant à un filtre apparié ; et
le dispositif de détection optique comprend en outre :
un processeur matériel (150) configuré pour déterminer un produit chimique candidat susceptible d'être présent dans la cible (152) sur la base de la comparaison de filtre apparié ; et
une interface de sortie configurée pour fournir une identification du produit chimique candidat en tant que sortie sur la base de la détermination.

12. Dispositif de détection optique selon la revendication 11, comprenant en outre une source de chaleur (140) configurée pour chauffer le capteur de test (128, 130, 132) à une température suffisante pour libérer le produit chimique de test (129, 131, 133) à partir du capteur de test (128, 130, 132).

13. Dispositif de détection optique selon la revendication 11 ou la revendication 12, comprenant en outre un dilatateur de faisceau (120) configuré pour dilater le faisceau optique afin de former un faisceau optique dilaté et un miroir d'orientation de faisceau (122) configuré pour orienter le faisceau optique dilaté, de sorte que le faisceau optique comprend une pluralité de faisceaux optiques déplacés spatialement au niveau de la cible (152).

14. Dispositif de détection optique selon l'une quelconque des revendications 11 à 13, comprenant en outre un dispositif de peigne d'infrarouge proche intégré (104) et/ou un dispositif de peigne d'infrarouge moyen intégré (106), où une sortie optique du dispositif de peigne d'infrarouge proche intégré (104) et du dispositif de peigne d'infrarouge moyen intégré (106) est fournie à l'émetteur (118).

15. Dispositif de détection optique selon l'une quelconque des revendications 11 à 14, dans lequel le réseau de détecteurs linéaire (144, 146) comprend en outre un dispositif de focalisation de faisceau (138) configuré pour recevoir le faisceau optique réfléchi ou diffusé en provenance de la cible (152), et un ou plusieurs miroirs de balayage (143) configurés pour fournir un ou plusieurs faisceaux optiques balayés au réseau de détecteurs linéaire (144, 146).
